# EUROPÄISCHE PATENTSCHRIFT

(11) **EP 1 280 791 B1**
(45) Veröffentlichungstag und Bekanntmachung des Hinweises auf die Patenterteilung: **03.05.2006**
(21) Anmeldenummer: 01273957.9
(22) Anmeldetag: 18.10.2001
(51) Int. Cl.: C07D 333/20, C07D 307/52, C07D 233/54, C07D 277/28, A61K 8/49, A61Q 5/10

(54) **N-HETEROARYLMETHYL-M-PHENYLENDIAMIN-DERIVATE ENTHALTENDE FÄRBEMITTEL FÜR KERATINFASERN SOWIE NEUE N-HETEROARYLMETHYL-M-PHENYLENDIAMIN-DERIVATE**
N-HETEROARYLMETHYL-M-PHENYLENEDIAMINE DERIVATIVE-CONTAINING DYES FOR KERATIN FIBERS AND NOVEL N-HETEROARYLMETHYL-M-PHENYLENEDIAMINE DERIVATIVES
COLORANTS CONTENANT DES DERIVES DE N-HETEROARYLMETHYLE-M-PHENYLENEDIAMINE POUR DES FIBRES DE KERATINE, AINSI QUE NOUVEAUX DERIVES DE N-HETEROARYLMETHYLE-M-PHENYLENEDIAMINE

(30) Priorität: 13.03.2001 DE 10111936
(43) Veröffentlichungstag der Anmeldung: 05.02.2003
(73) Patentinhaber: Wella Aktiengesellschaft, 64274 Darmstadt (DE)
(72) Erfinder: CHASSOT, Laurent, CH-1724 Praroman (CH); BRAUN, Hans-Jürgen, CH-3182 Überstorf (CH)
(86) Internationale Anmeldenummer: PCT/EP2001/012053
(87) Internationale Veröffentlichungsnummer: WO 2002/072568

(56) Entgegenhaltungen:
- EP-A- 0 024 660
- EP-A- 0 761 214
- EP-A- 0 963 982
- DE-A- 2 827 658

## Beschreibung

Die vorliegende Erfindung betrifft Mittel zur oxidativen Färbung von Keratinfesern, insbesondere menschlichen Haaren, auf der Basis einer Entwickler-substanz/Kupplersubstanz-Kombination, welche als Kupplersubstanz N-Heteroarylmothyl-m-phenylendiamin-Derivate enthalten, sowie neue N-Heteroarylmethyl-m-phanylendiamin-Derivate.

Auf dem Gebiet der Färbung von Keratinfasem, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophinylethylalkohol, p-Aminophenol, 1,4-Diaminobenzol und 4,5-Diamino-1-(2-hydroxyethyl)-pyrazol eingesetzt, während als Kupplersubstanzen beispielsweise Resorcin, 2-Methylresorcin, 1-Naphthol, 3-Aminophenol, m-Phenylendiamin, 2-Amino-4-(2'-hydroxyethyl)amino-anisol, 1,3-Diamino-4-(2'-hydroxyethoxy)benzol und 2,4-Diamino-5-fluor-totuol zu nennen sind.

An Oxidationsfarbstoffe, die zur Färbung menschlicher Haare verwendet werden, werden neben der Färbung in der gewünschten Intensität zahlreiche zusätzliche Anforderungen gestellt. So müssen die Farbstoffe in toxikologischer und dermatologischer Hinsicht unbedenklich sein und die erzielten Haarfärbungen eine gute Lichtechtheit, Dauerwellechtheit, Säureechtheit und Reibeechtheit aufweisen. Auf jeden Fall aber müssen solche Färbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwicklersubstanzen und Kupplersubstanzen eine breite Palette verschiedener Farbnuancen erzeugt werden kann.

Es wurde bereits versucht, die Eigenschaften von m-Phenyldiaminen durch die Einführung von Substituenten zu verbessern, In diesem Zusammenhang sei auf die DE-OS 29 34 330 verwiesen, in der unter anderem auch Färbemittel beschrieben werden, welche als Kupplersubstanzen spezielle N-substituierte m-Phenylendiamine enthalten. Mit den derzeit bekannten Färbemitteln ist es jedoch nicht möglich, die an ein Färbemittel gestellten Anforderungen in allen Punkten zu erfüllen.

Es bestand daher weiterhin ein Bedürfnis nach neuen Kupplersubstanzen, welche die vorgenannten Anforderung in besonderem Masse erfüllen.

Es wurde nunmehr gefunden, dass spezielle N-Heteroarylmethyl-m-phenylendiamin-Derivate der allgemeinen Formel (I) intensive stabile violette bis dunkelblaue Farbnuancen ermöglichen.

Gegenstand der vorliegenden Erfindung ist daher ein Mittel zur oxidativen Färbung von Keratinfasem, wie zum Beispiel Wolle, Pelzen, Federn oder Haaren, insbesondere menschlichen Haaren, auf der Basis einer Entwicklersubstanz-Kupplersubstanz-Kombination, das als Kupplersubstanz mindestens ein N-Heteroarylmethyl-m-phenylendiamin-Derivat der Formel (I) oder dessen Salz mit einer organischen oder anorganischen Säure enthält, 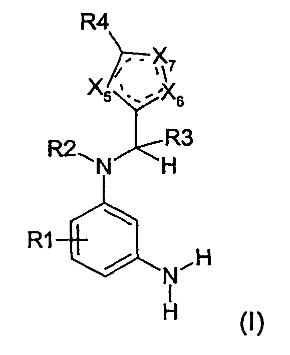 worin
**X5** gleich Schwefel, Stickstoff, Sauerstoff, C-R6 oder N-R5 ist;
**X6** gleich Schwefel, Stickstoff, Sauerstoff, C-R7 oder N-R5 ist
**X7** gleich Schwefel, Stickstoff, Sauerstoff, C-R8 oder N-R5 ist;
**R1** gleich Wasserstoff, einer C₁-C₄-Alkylgruppe, eine C₁-C₄-Hydroxyalkoxygruppe oder einer C₁-C₄-Hydroxyalkylgruppe ist;
**R2** und **R3** gleich oder verschieden sein können und unabhängig voneinander gleich Wasserstoff oder einer C₁-C₈-Alkylgruppe sind;
**R4, R6, R 7, R8** gleich oder verschieden sein können und unabhängig voneinander gleich Wasserstoff, einem Halogenatom (F, Cl, Br, J), einer Cyanogruppe, einer C₁-C₄-Alkoxygruppe, einer C₁-C₆-Alkylgruppe, einer C₁-C₄-Alkylthioethergruppe, einer Mercaptogruppe, einer Nitrogruppe, einer Aminogruppe, einer C₁-C₄-Alkylaminogruppe, einer Di(C₁-C₄)-alkylaminogruppe, einer Di(C₁-C₄-hydroxyalkyl)aminogruppe, einer C₁-C₄₋Hydroxyalkylaminogruppe, einer Trifluormethangruppe, einer -C(O)CH₃ Gruppe, einer -C(O)CF₃-Gruppe, einer -Si(CH₃)₃-Gruppe, einer C₁-C₄ Hydroxyalkylgruppe oder einer C₃-C₄-Dihydroxyalkylgruppe sind; und
**R5** gleich Wasserstoff, einer C₁-C₆-Alkylgruppe, einer C₂-C₄-Hydroxyalkylgruppe, einer Phenylgruppe oder einer Acetylgruppe ist; wobei mindestens einer und maximal zwei der Reste **X5** bis **X7** gleich C-R6 beziehungsweise C-R7 beziehungsweise C-R8 ist und maximal einer der Reste **X5** bis **X7** gleich Schwefel, Sauerstoff oder N-R5 ist.

Als Verbindungen der Formel (I) können beispielweise genannt werden: N-Thiophen-3-ylmethyl-1,3-diamino-benzol, N-Furan-3-ylmethyl-1,3-diamino-benzol, N-(1H-Imidazol-2-ylmethyl)-1,3-diamino-benzol, N-(1H-Pyrol-2-ylmethyl)-1,3-diamino-benzol, N-Thiophen-2-ylmethyl-1,3-diamino-benzol, N-Thiazol-2-ylmethyl-1,3-diamino-benzol, N-(5-Nitrothiophen-2-ylmethyl)-1,3-diamino-benzol, N-(3-Methyl-thiophen-2-ylmethyl)-1,3-diamino-benzol, N-(2-Methyl-thiophen-3-ylmethyl)-1,3-diamino-benzol, N-(4-Methyl-thiophen-3-ylmethyl)-1,3-diamino-benzol, N-(5-Methyl-thiophen-2-ylmethyl)-1,3-diamino-benzol, N-(3-Chlorthiophen-2-ylmethyl)-1,3-diamino-benzol, N-(4-Methyl-thiophen-2-ylmethyl)-1,3-diamino-benzol, N-(4-Chlor-thiophen-2-ylmethyl)-1,3-diamino-benzol, N-(5-Methyl-thiophen-2-ylmethyl)-1,3-diamino-benzol, N-(5-Chlor-thiophen-2-ylmethyl)-1,3-diamino-benzol, 2-{4-Amino-2-[(furan-2-ylmethyl)-amino]-phenoxy}-ethanol, 2-{4-Amino-2-[(thiophen-3-ylmethyl) -amino]-phenoxy}-ethanol, 2-{4-Amino-2-[(thiophen-2-ylmethyl)-amino]-phenoxy}-ethanol, 2-{4-Amino-2-[(1H-imidazol-2-ylmethyl}-amino]-phenoxyl-ethanol, 2-{4-Amino-2-[(furan-3-ylmethyl)-amino]-phenoxy}-ethanol, 2-{4-Amino-2-[(1H-pyrol-2-ylmethyl)-amino]-phenoxy}-ethanol, 2-{4-Amino-2-[(3-methyl-thiophen-2-ylmethyl)-amino]-phenoxy}-ethanol, 2-{4-Amino-2-[(3-chlor-thiophen-2-ylmethyl)-amino]-phenoxy}-ethanol, 2-{4-Amino-2-[(4-methyl-thiophen-2-ylmethyl)-amino]-phenoxy}-ethanol, 2-{4-Amino-2-[(4-chlor-thiophen-2-ylmethyl)-amino]-phenoxy}-ethanol, 2-{4-Amino-2-[(5-chlor-thiophen-2-ylmethyl)-amino]-phenoxy}-ethanol, 2-{4-Amino-2-[(5-methyl-thiophen-2-ylmethyl)-amino]-phenoxy}-ethanol, 2-{4-Amino-2-[(2-methyl-thiophen-3-ylmethyl)-amino]-phenoxy}-ethanol, 2-{4-Amino-2-[(2-chlor-thiophen-3-ylmethyl)-amino]-phenoxy}-ethanol, 2-{4-Amino-2-[(4-methyl-thiophen-3-ylmethyl)-amino]-phenoxy}-ethanol, 2-{4-Amino-2-[(4-chlor-thiophen-3-ylmethyl)-amino]-phenoxy}-ethanol, 2-{4-Amino-2-[(5-methyl-thiophen-3-ylmethyl)amino]-phenoxy}-ethanol, 2-{4-Amino-2-[(5-chlor-thiophen-3-ylmethyl)-amino]-phenoxy}-ethanol, 2-{2-Amino-4-[(furan-2-ylmethyl)-amino]-phenoxy}-ethanol, 2-{2-Amino-4-[(thiophen-3-ylmethyl)-amino]-phenoxy}-ethanol, 2-{2-Amino-4-[(thiophen-2-ylmethyl)-amino]-phenoxy}-ethanol, 2-{2-Amino-4-[(1H-imidazol-2-ylmethyl)-amino]-phenoxy}-ethanol, 2-{2-Amino-4-[(furan-3-ylmethyl)-amino]-phenoxy}-ethanol.

Bevorzugt sind Verbindungen der Formel (I), in denen (i) **R1** gleich Wasserstoff oder eine Hydroxyalkoxygruppe ist und R2 und R3 oder R2, R3 und R4 Wasserstoff bedeuten; oder (ii) R1 gleich Wasserstoff oder eine Hydroxyalkoxygruppe ist, die Reste R2 und R3 oder die Reste R2, R3 und **R4** Wasserstoff bedeuten, **X5** gleich Schwefel oder Sauerstoff ist, **X6** gleich Stickstoff oder C-R7 ist und **X7** gleich C-R8 ist, wobei mindestens einer der Reste **R7** und **R8** gleich Wasserstoff ist; oder (iii) **R1** gleich Wasserstoff oder eine Hydroxyalkoxygruppe ist, die Reste **R2** und **R3** oder die Reste **R2, R3** und **R4** gleich Wasserstoff sind, **X7** gleich Schwefel oder Sauerstoff ist, **X5** gleich C-R6 und **X6** gleich C-R7 ist, wobei mindestens einer der Reste R6 und R7 gleich Wasserstoff ist; oder (iv) **R1** gleich Wasserstoff oder eine Hydroxyalkoxygruppe ist, die Reste **R2** und **R3** oder die Reste **R2, R3** und **R4** gleich Wasserstoff sind, **X6** gleich Schwefel oder Sauerstoff ist, **X5** gleich C-R6 und **X7** gleich C-R8 ist, wobei mindestens einer der Reste **R6** und **R8** gleich Wasserstoff ist.

Besonders bevorzugt sind die folgenden N-Heteroarylmethyl-m-phenylendiamin-Derivate der Formel (I): N-Thiophen-3-ylmethyl-1,3-diamino-benzol, N-Furan-3-ylmethyl-1,3-diamino-benzol, N-Furan-2-ylmethyl-1,3-diamino-benzol, N-Thiophen-2-ylmethyl-1,3-diamino-benzol, 2-{4-Amino-2-[(thiophen-3-ylmethyl)-amino]-phenoxy}-ethanol, 2-{4-Amino-2-[(thiophen-2-ylmethyl)-amino]-phenoxy}-ethanol, 2-{4-Amino-2-[(furan-3-ylmethyl)-amino]-phenoxy}-ethanol, 2-{4-Amino-2-[(furan-2-ylmethyl)-amino]-phenoxy}-ethanol, 2-{2-Amino-4-[(thiophen-2-ylmethyl)-amino]-phenoxy}-ethanol und 2-{2-Amino-4-[(furan-2-ylmethyl)-amino]-phenoxy}-ethanol oder deren Salze.

Die Verbindungen der Formel (I) können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Die N-Heteroarylmethyl-m-phenylendiamin-Derivate der Formel (I) sind in dem erfindungsgemäßen Färbemittel in einer Gesamtmenge von etwa 0,005 bis 20 Gewichtsprozent enthalten, wobei eine Menge von etwa 0,01 bis 5 Gewichtsprozent und insbesondere 0,1 bis 2,5 Gewichtsprozent bevorzugt ist.

Als Entwicklersubstanzen können alle für derartige Färbemittel bekannten und geeigneten Entwicklersubstanzen, beispielsweise 1,4-Diaminobenzol (p-Phenylendiamin), 1,4-Diamino-2-methyl-benzol (p-Toluylendiamin), 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 1,4-Diamino-2-(pyridin-3-yl)benzol, 2,5-Diaminobiphenyl, 1,4-Diamino-2-rnethoxymethyl-benzol, 1,4-Diamino-2-aminomethyl-benzol, 1,4-Diamino-2-hydroxymethyl-benzol, 1,4-Diamino-2-(2-hydroxyethoxy)-benzol, 1-(2,5-Diamino-phenyl)-ethanol, 2-(2-(Acetylamino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)amino]-anilin, 4-[Di(2-hydroxyethyl)amino]-anilin, 4-[Di(2-hydroxyethyl)amino]-2-methyl-anilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluor-phenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino)methylphenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1H-pyrazol, 4,5-Diamino-1-[(4-methylphenyl)methyl]-1H-pyrazol, 1-[(4-Chlorphenyl)methyl]-4,5-diamino-1H-pyrazol, 4,5-Diamino-1-methyl-1H-pyrazol, 4,5-Diamino-3-methyl-1-methyl-1H-pyrazol, 4,5-Diamino-3-methyl-1-methyl-1H-pyrazol, 2-Amino-phenol, 2-Amino-6-methyl-phenol und 2-Amino-5-methyl-phenol, eingesetzt werden.

Weiterhin kann das erfindungsgemäße Färbemittel gegebenenfalls zusätzlich weitere bekannte Kupplersubstanzen, beispielsweise 2,6-Diamino-pyridin, 2-Amino-4-[((2-hydroxyethyl))amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxybenzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methylphenol, 5-Amino-4-methoxy-2-methyl-phenol, 5-Amino-4-ethoxy-2-methylphenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methylphenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol,7-Hydroxy-indol und 2,3-Indolindion, enthalten.

Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemäßen Färbemittel (bezogen auf die Gesamtmenge des Färbemittels) jeweils etwa 0,005 bis 20 Gewichtsprozent, vorzugsweise etwa 0,01 bis 5,0 Gewichtsprozent und insbesondere 0,1 bis 2,5 Gewichtsprozent, beträgt. Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6 Gewichtsprozent besonders bevorzugt ist. Die Entwicklersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Überschuß oder Unterschuß (beispielsweise in einem Verhältnis (Kuppler : Entwickler) von 1:2 bis 1:0,5) vorhanden sind.
Weiterhin kann das erfindungsgemäße Färbemittel zusätzlich andere Farbkomponenten, beispielsweise 6-Amino-2-methylphenol und 2-Amino-5-methylphenol, sowie femer übliche direktziehende Farbstoffe, zum Beispiel Triphenylmethanfarbstoffe wie 4-[(4'-aminophenyl)-(4'imino-2",5"-cyclohexadien-1"-yliden)-methyl]-2-methylaminobenzol-monohydrochlorid (C.l. 42 510) und 4-[(4'amino-3'-methyl-phenyl)-(4"-imino-3"-methyl-2",5"cyclohexadien-1"-yliden)-methyl]-2-methyl-aminobenzol monohydrochlorid (C.l. 42 520), aromatische Nitrofarbstoffe wie 4-(2'-hydroxyethyl)amino-nitrotoluol, 2-Amino-4,6-dinitrophenol, 2-Amino-5-(2'-hydroxyethyl)amino-nitrobenzol, 2-Chlor-6-(ethylamino)-4-nitrophenol, 4-Chlor-N-(2-hydroxyethyl-2-nitroanilin, 5-Chlor-2-hydroxy-4-nitroanilin, 2-Amino-4-chlor-6-nitrophenol und 1-[(2'-Ureidoethyl)amino-4-nitrobenzol, Azofarbstoffe wie 6-[(4'-Aminophenyl)azo]-5-hydroxynaphthalin-1-sulfonsäure-Natriumsalz (C.l. 14 805) und Dispersionsfarbstoffe wie beispielsweise 1,4-Diaminoanthrachinon und 1,4,5,8-Tetraamino-antrachinon, enthalten. Die vorgenannten Farbkomponenten können in dem erfindungsgemäßen Färbemittel in einer Menge von etwa 0,1 bis 4 Gewichtsprozent enthalten sein.

Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemäßen Färbemittel (bezogen auf die Gesamtmenge des Färbemittels) jeweils etwa 0,005 bis 20 Gewichtsprozent, vorzugsweise etwa 0,01 bis 5,0 Gewichtsprozent und insbesondere 0,1 bis 2,5 Gewichtsprozent, beträgt. Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6 Gewichtsprozent besonders bevorzugt ist. Die Entwicklersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Überschuß oder Unterschuß (beispielsweise in einem Verhältnis (Kuppler : Entwickler) von 1:2 bis 1:0,5) vorhanden sind.

Weiterhin kann das erfindungsgemäße Färbemittel zusätzlich andere Farbkomponenten, beispielsweise 6-Amino-2-methylphenol und 2-Amino-5-methylphenol, sowie femer übliche anionische, kationische, zwitterionische oder nicht-ionische direktziehende Farbstoffe, beispielsweise Triphenylmethanfarbstoffe wie 4-[(4'-aminophenyl)-(4'imino-2",5"-cyclohexadien-1"-yliden)-methyl]-2-methylaminobenzol-monohydrochlorid (C.I. 42 510) und 4-[(4'amino-3'-methyl-phenyl)-(4"-imino-3"-methyl-2",5"cyclohexadien-1"-yliden)methyl]-2-methyl-aminobenzol monohydrochlorid (C.I. 42 520), aromatische Nitrofarbstoffe wie 4-(2'-hydroxyethyl)amino-nitrotoluol, 2-Amino-4,6-dinitrophenol, 2-Amino-5-(2'-hydroxyethyl)amino-nitrobenzol, 2-Chlor-6-(ethylamino)-4-nitro-phenol, 4-Chlor-N-(2-hydroxyethyl-2-nitroanilin, 5-Chlor-2-hydroxy-4-nitroanilin, 2-Amino-4-chlor-6-nitrophenol und 1-[(2'-Ureidoethyl)amino-4-nitrobenzol, Azofarbstoffe wie 6-[(4'-Aminophenyl)azo]-5-hydroxy-naphthalin-1-sulfonsäure-Natriumsalz (C.I. 14 805) und Dispersionsfarbstoffe wie beispielsweise 1,4-Diaminoanthrachinon und 1,4,5,8-Tetraamino-antrachinon, enthalten. Die vorgenannten Farbkomponenten und direktziehenden Farbstoffe können in dem erfindungsgemäßen Färbemittel jeweils in einer Menge von etwa 0,1 bis 4 Gewichtsprozent enthalten sein.

Selbstverständlich können die Kupplersubstanzen und Entwickler substanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in dem erfindungsgemäßen Färbemittel, falls dieses zur Färbung von Haaren verwendet werden soll, noch weitere für kosmetische Mittel übliche Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.

Die Zubereitungsform des erfindungsgemäßen Färbemittels kann beispielsweise eine Lösung, insbesondere eine wässrige oder wässrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsutfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie hohere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5 Gewichtsprozent.

Das gebrauchsfertige erfindungsgemäße Haarfärbemittel wird durch Mischen des Färbemittels mit einem Oxidationsmittel unmittelbar vor der Anwendung hergestellt.

Als Oxidationsmittel kommen hauptsächlich Wasserstoffperoxid oder dessen Additionsverbindungen an Harnstoff, Melamin, Natriumborat oder Natriumcarbonat in Form einer 1- bis 12prozentigen, vorzugsweise einer 3- bis 6prozentigen, wässrigen Lösung, in Betracht. Das Gewichtsverhältnis zwischen Haarfärbemittel und Oxidationsmittel beträgt hierbei vorzugsweise etwa 5:1 bis 1:3, insbesondere 1:1 bis 1:2. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Es ist jedoch prinzipiell auch möglich, zur Oxidation der Farbstoffe anstelle der vorgenannten Oxidationsmittel Luftsauerstoff zu verwenden.

Des pH-Wert des gebrauchsfertigen erfindungsgemäßen Haarfärbemittels stellt sich bei der Mischung des Färbemittels (dessen pH-Wert etwa gleich 6 bis 11,5 ist) mit dem meist sauer eingestellten Oxidationsmittel (dessen pH-Wert etwa gleich 2 bis 6,5 ist) auf einen pH-Wert ein, der durch die Alkalimengen in dem Färbemittel und die Säuremengen im Oxidationsmittel sowie durch das Mischungsverhältnis bestimmt wird. Je nach Zusammensetzung kann das erfindungsgemäße Färbemittel schwach sauer, neutral oder alkalisch reagieren und im gebrauchsfertigen Zustand einen pH-Wert von etwa 3 und 11,5, vorzugsweise etwa 6 bis 10, aufweisen. Die basische Einstellung erfolgt hierbei vorzugsweise mit Ammoniak, wobei jedoch auch organische Amine, zum Beispiel 2-Amino-2-methyl-1-propanol, Tris(hydroxymethyl)amino-methan, Monoethanolamin und Triethanolamin, oder auch anorganische Basen wie Natriumhydroxid und Kaliumhydroxid Verwendung finden. Für eine pH-Wert-Einstellung im sauren Bereich kommen anorganische oder organische Säuren, zum Beispiel Phosphorsäure, Essigsäure, Milchsäure, Ascorbinsäure, Zitronensäure oder Weinsäure, in Betracht.

Anschliessend trägt man eine für die Haarfärbebehandlung ausreichende Menge, je nach Haarfülle, im allgemeinen etwa 60 bis 200 Gramm, dieses Gemisches auf das Haar auf und läßt das Gemisch bei etwa 15 bis 50 Grad Celsius, vorzugsweise 30 bis 40 Grad Celsius, etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Das erfindungsgemäße Färbemittel mit einem Gehalt an N-Heteroaryl-methyl-m-phenylendiamin-Derivaten der Formel (I) als Kupplersubstanz ermöglicht Färbungen mit ausgezeichneter Farbechtheit, insbesondere was die Lichtechtheit, Waschechtheit und Reibeechtheit anbetrifft. Hinsichtlich der färberischen Eigenschaften bietet das erfindungsgemäße Färbemittel je nach Art und Zusammensetzung der Farbkomponenten eine breite Palette verschiedener Farbnuancen, welche sich von blonden über braune, purpume, violette bis hin zu blauen und schwarzen Farbtönen erstreckt, wobei insbesondere die erzielbaren violetten bis dunkelblauen Farbtöne hervorzuheben sind. Hierbei zeichnen sich die Farbtöne durch ihre besondere Farbintensität aus. Die sehr guten färberischen Eigenschaften des Färbemittels gemäß der vorliegenden Anmeldung zeigen sich weiterhin darin, dass dieses Mittel insbesondere auch eine Anfärbung von ergrauten, chemisch nicht vorgeschädigten Haaren problemlos und mit guter Deckkraft ermöglicht.

Die Herstellung der erfindungsgemäßen N-Heteroarylmethyl-m-phenylendiamin-Derivate der Formel (I) kann unter Verwendung von bekannten Syntheseverfahren, beispielsweise dem in den Ausführungsbeispielen beschriebenen Verfahren, erfolgen.

Die N-Heteroarylmethyl-m-phenylendiamin-Derivate der Formel (I) sind gut in Wasser löslich und ermöglichen Färbungen mit hoher Farbintensität und ausgezeichneter Farbechtheit, insbesondere was die Lichtechtheit, Waschechtheit und Reibeechtheit anbetrifft. Sie weisen weiterhin eine ausgezeichnete Lagerstabilität, insbesondere als Bestandteil der hier beschriebenen Oxidationsfärbemitteln, auf.

Ein weiterer Gegenstand der vorliegenden Erfindung sind N-Heteroaryl-methyl-m-phenylendiamin-Derivate der allgemeinen Formel (II) oder deren wasserlösliche Salze mit organischen oder anorganischen Säuren, 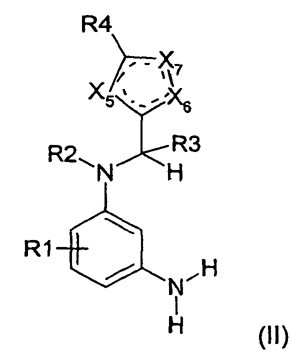 worin
**X5** gleich Schwefel, Stickstoff, Sauerstoff, C-R6 oder N-R5 ist;
**X6** gleich Schwefel, Stickstoff, Sauerstoff, C-R7 oder N-R5 ist
**X7** gleich Schwefel, Stickstoff, Sauerstoff, C-R8 oder N-R5 ist;
**R1** gleich Wasserstoff, einer C₁-C₄-Alkylgruppe, eine C₁-C₄-Hydroxyalkoxygruppe oder einer C₁-C₄-Hydroxyalkylgruppe ist;
**R2** und **R3** gleich oder verschieden sein können und unabhängig voneinander gleich Wasserstoff oder einer C₁-C₈-Alkylgruppe sind;
**R4, R6, R 7, R8** gleich oder verschieden sein können und unabhängig voneinander gleich Wasserstoff, einem Halogenatom (F, Cl, Br, J), einer Cyanogruppe, einer C₁-C₄-Alkoxygruppe, einer C₁-C₆-Alkylgruppe, einer C₁-C₄-Alkylthioethergruppe, einer Mercaptogruppe, einer Nitrogruppe, einer Aminogruppe, einer C₁-C₄-Alkylaminogruppe, einer Di(C₁-C₄)-alkylaminogruppe, einer Di(C₁-C₄-hydroxyalkyl)aminogruppe, einer C₁-C₄₋Hydroxyalkylaminogruppe, einer Trifluormethangruppe, einer -C(O)CH₃ Gruppe, einer -C(O)CF₃-Gruppe, einer -Si(CH₃)₃-Gruppe, einer C₁-C₄ Hydroxyalkylgruppe oder einer C₃-C₄-Dihydroxyalkylgruppe sind; und
**R5** gleich Wasserstoff, einer C₁-C₆-Alkylgruppe, einer C₂-C₄-Hydroxyalkylgruppe, einer Phenylgruppe oder einer Acetylgruppe ist; wobei mindestens einer und maximal zwei der Reste X5 bis X7 gleich C-R6 beziehungsweise C-R7 beziehungsweise C-R8 ist und maximal einer der Reste **X5** bis **X7** gleich Schwefel, Sauerstoff oder N-R5 ist;
unter den Bedingungen, dass mindestens einer der Reste **R1**, **R2, R3, R4, R6, R7** und **R8** nicht gleich Wasserstoff ist wenn **X5** oder **X6** gleich Schwefel ist.

Die nachfolgenden Beispiele sollen den Gegenstand der Erfindung näher erläutern, ohne ihn darauf zu beschränken.

### Beispiele

### Beispiel 1: Synthese von N-Heteroarylmethyl-1,3-diamino-benzolen

### A. Synthese von (3-Amino-phenyl)-carbaminsäure-tert.butylester

Zu einer Lösung von 10,8 g (100 mmol) 1,3-Phenylendiamin in 100 ml Methylenchlorid/NaOH(4%) 1:1 wird eine Lösung von 11 g (50 mmol) Di-tert.-butyl-dicarbonat in 30 ml Methylenchlorid zugetropft. Das Reaktionsgemisch wird 8 Stunden lang gerührt und sodann mit 5 g Di-tert.-butyl-dicarbonat versetzt. Anschließend wird die Reaktionsmischung weitere 12 Stunden bei Raumtemperatur gerührt. Die organische Phase wird anschliessend mit einer gesättigten wässerigen Kochsalz-Lösung gewaschen, über Natriumsulfat getrocknet und nach Filtration eingeengt. Das erhaltene Rohprodukt wird an Kieselgel mit Hexan/Essigsäureethylester (1:1) gereinigt. Es werden 6,2 g (30% der Theorie) (3-Aminophenyl)-carbaminsäure-tert.butylester erhalten. ¹H-NMR (300MHz, CDCl₃): δ = 7,06 (t, 1H); 6,99 (br s, 1H); 6,44 (br s, 2H); 6,56 (d, 1H); 6,39 (m, 2H); 3,68 (br, 2H); 1,53 (s, 9H)

### B. Synthese von N-Heteroarylmethyl-1,3-diamino-benzolen

0,031 g (0,15 mmol) (3-Aminophenyl)-carbaminsäure-tert-butylester aus Stufe **1A** und 0,1 mmol des entsprechenden Aldehyds werden in Methanol (über Molekularsieb getrocknet) gelöst. 10 mg Molekülarsieb wird zugegeben und das Reaktionsgemisch wird für 7 Stunden gerührt. Anschließend werden bei 0 °C 0,3 ml einer BoranTetrahydrofuran-Komplexlösung (1 M in Tetrahydrofuran) zugegeben und die Reaktionsmischung eine Stunde lang bei Raumtemperatur gerührt. Nach Beendigung der Reaktion wird die Reaktionsmischung in Wasser gegossen und mit Essigsäureethylester extrahiert. Die organische Phase wird mit Magnesiumsulfat getrocknet, das Lösungsmittel im Rotationsverdampfer abdestilliert und der Rückstand an Kieselgel mit Petrolether/Essigsäureethylester (1:1) gereinigt. Das so erhaltene Produkt wird in 4 ml Ethanol und 1,5 ml einer 2,9molaren ethanolische Salzsäurelösung auf 50 °C erwärmt. Der erhaltene Niederschlag wird abfiltriert, zweimal mit 1 ml Ethanol gewaschen und sodann getrocknet.
**a**. N-Thiophen-2-ylmethyl-1,3-diamino-benzol Hydrochlorid
   Verwendetes Aldehydderivat: Thiophen-2-carbaldehyd
   Massenspektrum: MH+ 203(100)
**b.** N-Thiophen-3-ylmethyl-1,3-diamino-benzol Hydrochlorid
   Verwendetes Aldehydderivat: Thiophen-3-carbaldehyd
   Massenspektrum: MH+ 205(100)
**c**. N-Furan-2-ylmethyl-1,3-diamino-benzol Hydrochlorid
   Verwendetes Aldehydderivat: Furan-2-carbaldehyd
   Massspketren: MH+ 189(100)
**d**. N-Furan-3-ylmethyl-1,3-diamino-benzol Hydrochlorid
   Verwendetes Aldehydderivat: Furan-3-carbaldehyd
   Massenspektrum: MH+ 189(100)
**e.** N-(1H-Imidazol-2-ylmethyl-1,3-diamino-benzol Hydrochlorid
   Verwendetes Aldehydderivat: Imidazol-2-carbaldehyd
   Massenspektrum: MH+ 189(100)
**f**. N-Thiazol-2-ylmethyl-1,3-diamino-benzol Hydrochlorid
   Verwendetes Aldehydderivat: Thiazol-2-carbaldehyd
   Massenspektrum: MH+ 242(100)
**g.** N-(5-Nitro-thiophen-2-ylmethyl)-1,3-diamino-benzol Hydrochlorid
   Verwendetes Aldehydderivat: 5-Nitro-thiophen-2-carbaldehyd
   Massenspektrum: MH+ 250(20)

### Beispiel 2: Synthese von 2-{4-Amino-2-[(N-heteroarylmethyl)-amino]-phenoxy}-ethanolen

### A. Synthese von [3-Amino-4-(2-hydroxy-ethoxy)-phenyl]-carbaminsäure-tert.butylester

Zu einer Lösung von 10,7 g (100 mmol) 2-(2,4-Diaminophenoxy)ethanol in 300 ml Acetonitril wird eine Lösung von 16,8 g NaHCO₃ in 100 ml Wasser getropft. Anschließend werden 22 g (100 mmol) Di-tert.-butyl-dicarbonat hinzugefügt und das Reaktionsgemisch 6 Stunden lang gerührt. Nach Beendigung der Reaktion wird die Reaktionsmischung in 100 ml Dichlormethan gegossen und die organische Phase mit verdünnter Salzsäure extrahiert. Die wässerige Phase wird mit einer gesättigten 2 N Natriumhydroxid-Lösung alkalisiert und anschliessend mit Dichlormethan extrahiert. Die organische Phase wird über Natriumsulfat getrocknet und sodann am Rotationsverdampfer abdestilliert. Es werden 10,3 g (38% der Theorie) [3-Amino-4-(2-hydroxy-ethoxy)-phenyl]-carbaminsäure-tert.butylester erhalten.
¹H-NMR (300MHz, CDCl₃) δ = 7,26 (br s, 1H); 6,95 (d, 1H); 6.72 (d, 1H); 6,34 (s, 1H); 4,77 (m, 2H); 3,9 (m, 2H); 1.50 (s, 9H).

### B. Synthese von 2-{4-Amino-2-[(N-heteroarylmethyl)-amino]-phenoxy}-ethanolen

0,031 g (0,15 mmol) [3-Amino-4-(2-hydroxy-ethoxy)-phenyl]-carbaminsäure-tert.butylester aus Beispiel **2A** und 0,1 mmol des entsprechenden Aldehyds werden in Methanol (über Molekularsieb getrocknet) gelöst. 10 mg Molekülarsieb wird zugegeben und das Reaktionsgemisch wird für 7 Stunden gerührt. Anschließend werden 0,3 ml einer BoranTetrahydrofuran-Komplexlösung (1 M in Tetrahydrofuran) bei 0 °C zugegeben und die Reaktionmischung eine Stunde lang bei Raumtemperatur gerührt. Nach Beendigung der Reaktion wird die Reaktionsmischung in Wasser gegossen und mit Essigsäureethylester extrahiert. Die organische Phase wird mit Magnesiumsulfat getrocknet. Das Lösungsmittel wird am Rotationsverdampfer abdestilliert und der Rückstand an Kieselgel mit Petrolether/Essigsäureethylester (1:1) gereinigt. Das so erhaltene Produkt wird in 4 ml Ethanol und 1,5 ml einer 2,9 molaren ethanolische Salzsäurelösung auf 50 °C erwärmt. Der Niederschlag wird abfiltriert, zweimal mit 1 ml Ethanol gewaschen und sodann getrocknet.
**a**. 2-{4-Amino-2-[(thiophen-2-ylmethyl)-amino]-phenoxy}-ethanol Hydrochlorid
   Verwendete Aldehydderivat: Thiophen-2-carbaldehyd
   Massenspketrum: MH+ 265(100)
**b.** 2-{4-Amino-2-[(furan-2-ylmethyl)-amino]-phenoxy}-ethanol Hydrochlorid
   Verwendete Aldehydderivat: Furan-2-carbaldehyd
   Massenspketrum:: MH+ 249(10)
**c**. 2-{4-Amino-2-[(thiophen-3-ylmethyl)-amino]-phenoxy}-ethanol Hydrochlorid
   Verwendete Aldehydderivat: Thiophen-3-carbaldehyd
   Massenspketrum: MH+ 265(20)
**d**. 2-{4-Amino-2-[(1H-imidazol-2-ylmethyl)-amino]-phenoxy}-ethanol Hydrochlorid
   Verwendete Aldehydderivat: lmidazol-2-carbaldehyd
   Massenspketrum: MH+ 249(10)
**e.** 2-{4-Amino-2-[(furan-3-ylmethyl)-amino]-phenoxy}-ethanol Hydrochlorid
   Verwendete Aldehydderivat: Furan-3-carbaldehyd
   Massenspketrum: MH+ 249(20)

### Beispiel 3 bis 14: Haarfärbemittel

Es werden Haarfärbelösungen der folgenden Zusammensetzung hergestellt:

| | |
|---|---|
| 1,25 mmol | Kupplersubstanz der Formel (I) gemäß Tabelle 1 |
| 1,25 mmol | Entwicklersubstanz gemäß Tabelle 1 |
| 1,0 g | Kaliumoleat (8prozentige wässrige Lösung) |
| 1,0 g | Ammoniak (22prozentige wässrige Lösung) |
| 1,0 g | Ethanol |
| 0,3 g | Ascorbinsäure |
| ad 100,0 g | Wasser |

50 g der vorstehenden Färbelösung werden unmittelbar vor der Anwendung mit 50 g einer 6prozentigen wässrigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die resultierenden Färbungen sind in Tabelle 1 zusammengefaßt.

**Tabelle 1:**

| **Beispiel Nr.** | **Kupplersubstanz der Formel (I)** | **Entwicklersubstanz** | | | |
|---|---|---|---|---|---|
| | | **I. 1,4-Diaminobenzol** | **II. 2,5-Diaminotoluolsulfat** | **III. 2,5-Diaminophenylethanolsulfat** | **IV. ,5-iamino-1-2'-hydroxythyl)-yrazol-ulfat** |
| **3.** | gemäß Beispiel **1a** | dunkelblau | dunkelblau | dunkelblau | violett |
| **4.** | gemäß Beispiel **1b** | dunkelblau | dunkelblau | dunkelblau | violett |
| **5.** | gemäß Beispiel **1c** | dunkelblau | dunkelblau | dunkelblau | violett |
| **6.** | gemäß Beispiel **1d** | dunkelblau | dunkelblau | dunkelblau | violett |
| **7.** | gemäß Beispiel **1e** | blau | blau | blau | violett |
| **8.** | gemäß Beispiel **1f** | blau | blau | blau | violett |
| **9.** | gemäß Beispiel **1g** | blau | graublau | graublau | hell-violett |
| **10.** | gemäß Beispiel **2a** | dunkelblau | dunkelblau | dunkelblau | violett |
| **11.** | gemäß Beispiel **2b** | dunkelblau | dunkelblau | dunkelblau | violett |
| **12.** | gemäß Beispiel **2c** | dunkelblau | dunkelblau | dunkelblau | violett |
| **13.** | gemäß Beispiel **2d** | dunkelblau | dunkelblau | dunkelblau | violett |
| **14.** | gemäß Beispiel **2e** | dunkelblau | dunkelblau | dunkelblau | violett |

### Beispiel 15 bis 39: Haarfärbemittel

Es werden Haarfärbelösungen der folgenden Zusammensetzung hergestellt:

| | |
|---|---|
| X g | N-Heteroaraylmethyl-1,3-diamino-benzol (Kupplersubstanz **K1** bis **K4** der Formel (I) gemäß Tabelle 3) |
| U g | Entwicklersubstanz **E8** bis **E15** gemäß Tabelle 2 |
| Y g | Kupplersubstanz **K11** bis **K36** gemäß Tabelle 3 |
| Z g | 6-Chlor-2-ethylamino-4-nitro-phenol (**D2**) |
| 10,0 g | Kaliumoleat (8prozentige wässrige Lösung) |
| 10,0 g | Ammoniak (22prozentige wässrige Lösung) |
| 10,0 g | Ethanol |
| 0,3 g | Ascorbinsäure |
| ad 100,0 g | Wasser |

30 g der vorstehenden Färbelösung werden unmittelbar vor der Anwendung mit 30 g einer 6prozentigen wässsrigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die Färbeergebnisse sind in Tabelle 4 zusammengefasst.

### Beispiel 39 bis 62: Haarfärbemittel

Es werden cremeförmige Farbträgermassen der folgenden Zusammensetzung hergestellt:

| | |
|---|---|
| X g | N-Heteroaraylmethyl-1,3-diamino-benzol (Kupplersubstanz **K1** bis **K4** der Formel (I) gemäß Tabelle 3) |
| U g | Entwicklersubstanz **E8** bis **E15** gemäß Tabelle 2 |
| Y g | Kupplersubstanz **K11** bis K36 gemäss Tabelle 3 |
| Z g | 6-Chlor-2-ethylamino-4-nitro-phenol (**D2**) |
| 15,0 g | Cetylalkohol |
| 0,3 g | Ascorbinsäure |
| 3,5 g | Natriumlaurylalkoholdiglycolethersulfat, 28prozentige wässrige Lösung |
| 3,0 g | Ammoniak, 22prozentige wässrige Lösung |
| 0,3 g | Natriumsulfit, wasserfrei |
| ad 100 g | Wasser |

30 g der vorstehenden Färbecreme werden unmittelbar vor der Anwendung mit 30 g einer 6prozentigen Wasserstoffperoxidlösung vermischt. Anschließend wird das Gemisch auf das Haar aufgetragen. Nach einer Einwirkzeit von 30 Minuten bei 40 °C wird das Haar mit Wasser gespült, mit einem handelsüblichen Shampoo gewaschen und getrocknet. Die Färbeergebnisse sind in Tabelle 5 zusammengefasst.

**Tabelle 2:**

| **Entwicklersubstanzen** | |
|---|---|
| **E8** | 1,4-Diaminobenzol |
| **E9** | 2,5-Diamino-phenylethanol-sulfat |
| **E10** | 3-Methyl-4-amino-phenol |
| **E11** | 4-Amino-2-aminomethyl-phenol*2HCl |
| **E12** | 4-Amino-phenol |
| **E13** | N,N-Bis(2'-hydroxyethyl)-p-phenylendiamin-sulfat |
| **E14** | 4,5-Diamino-1-(2'-hydroxyethyl)-pyrazol-sulfat |
| **E15** | 2,5-Diaminotoluol-sulfat |

**Tabelle 3:**

| **Kupplersubstanzen** | |
|---|---|
| **K1** | N-Thiophen-2-ylmethyl-1,3-diamino-benzol*HCl |
| **K2** | N-Furan-2-ylmethyl-1,3-diamino-benzol*HCl |
| **K3** | 2-{4-Amino-2-[(thiophen-2-ylmethyl)-amino]-phenoxy}-ethanol*HCl |
| **K4** | 2-{4-Amino-2-[(furan-2-ylmethyl)-amino]-phenoxy}-ethanol*HCl |
| | |
| **K11** | 1,3-Diaminobenzol |
| **K12** | 2-Amino-4-(2'-hydroxyethyl)amino-anisol-sulfat |
| **K13** | 1,3-Diamino-4-(2'-hydroxyethoxy)benzol-sulfat |
| **K14** | 2,4-Diamino-5-fluor-toluol-sulfat |
| **K15** | 3-Amino-2-methylamino-6-methoxy-pyridin |
| **K16** | 3,5-Diamino-2,6-dimethoxy-pyridin*2HCl |
| **K17** | 2,4-Diamino-5-ethoxy-toluol-sulfat |
| **K18** | N-(3-Dimethylamino)phenylhamstoff |
| **K19** | 1,3-Bis(2,4-Diaminophenoxy)propan*4HCl |
| **K21** | 3-Amino-phenol |
| **K22** | 5-Amino-2-methyl-phenol |
| **K23** | 3-Amino-2-chlor-6-methyl-phenol |
| **K24** | 5-Amino-4-fluor-2-methyl-phenol-sulfat |
| **K25** | 1-Naphthol |
| **K26** | 1-Acetoxy-2-methyl-naphthalin |
| **K31** | 1,3-Dihydroxy-benzol |
| **K32** | 2-Methyl-1,3-dihydroxy-benzol |
| **K33** | 1-Chlor-2,4-dihydroxy-benzol |
| **K34** | 4-(2'-Hydroxyethyl)amino-1,2-methylendioxybenzol*HCl |
| **K35** | 3,4-Methylendioxy-phenol |
| **K36** | 2-Amino-5-methyl-phenol |

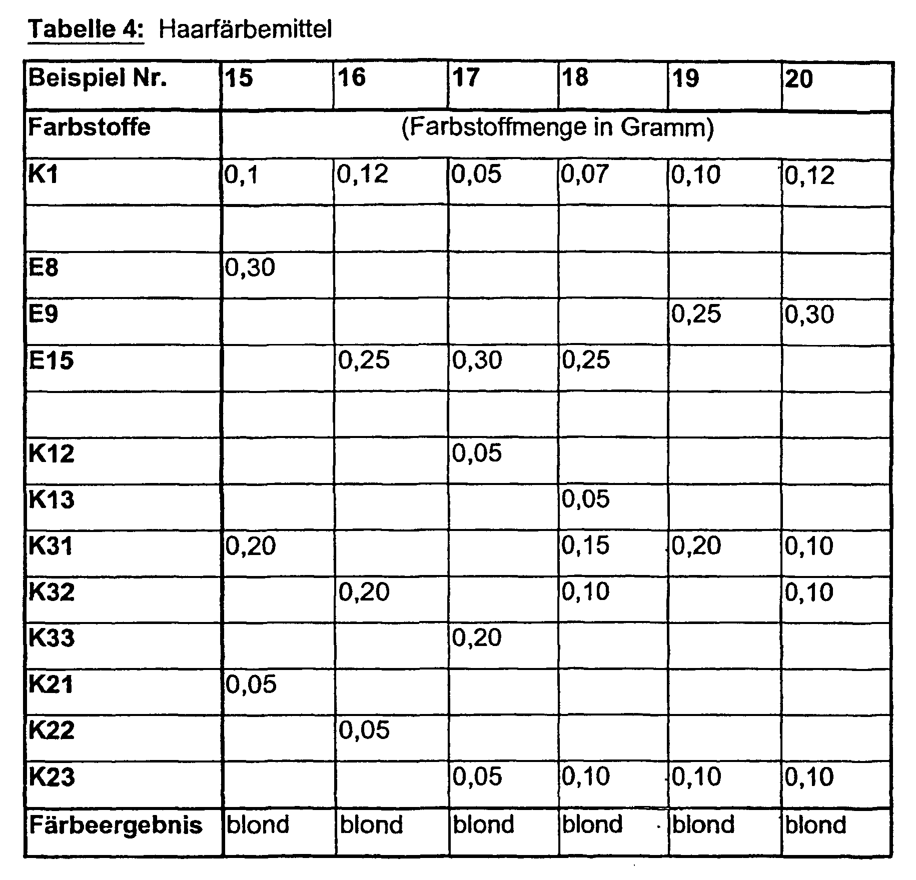

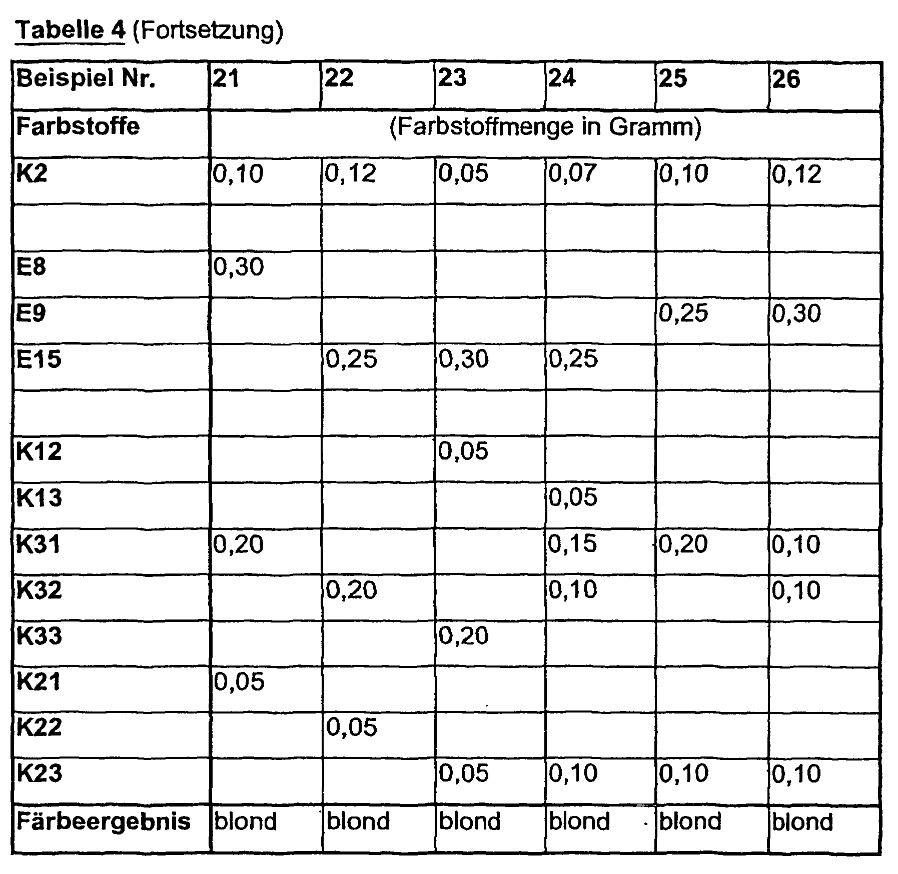

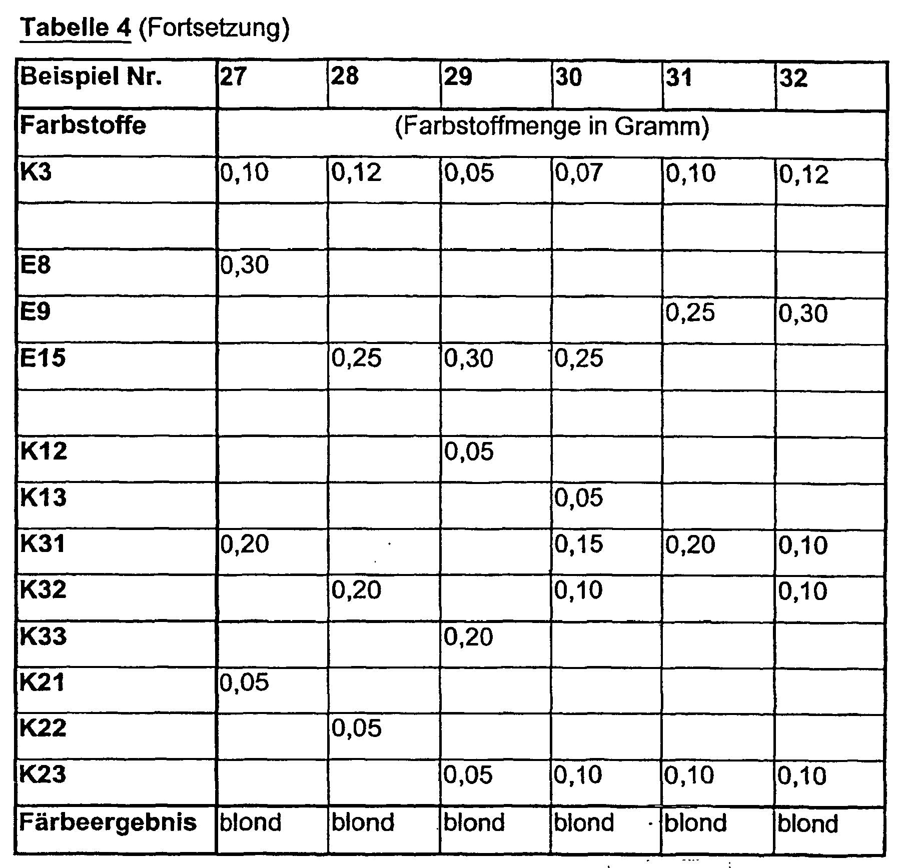

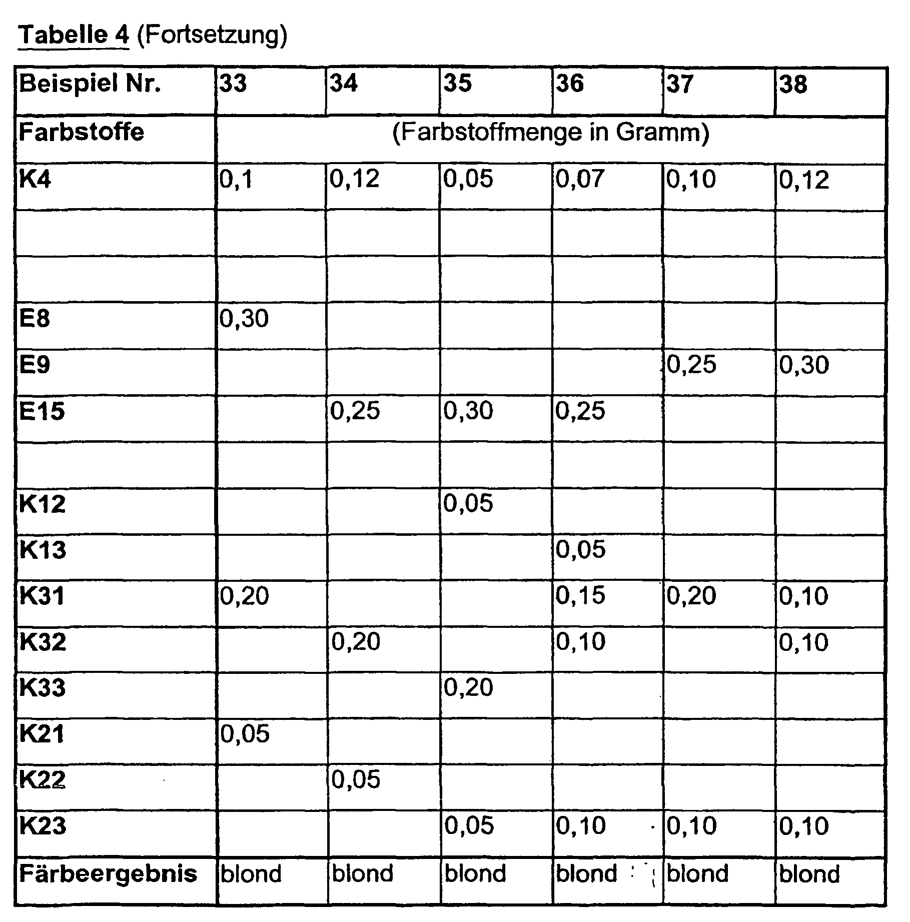

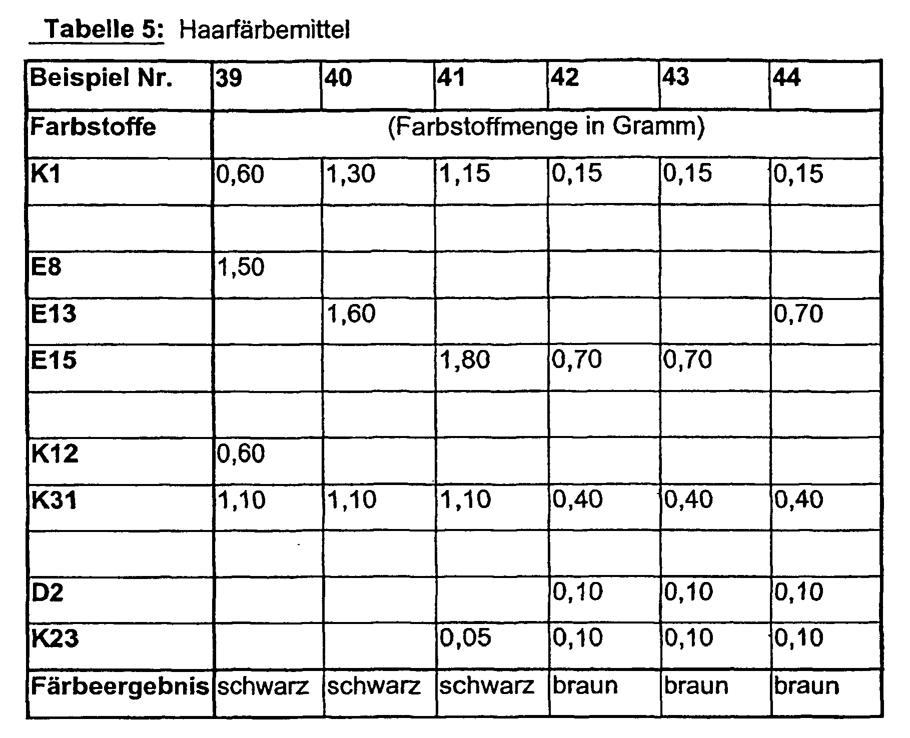

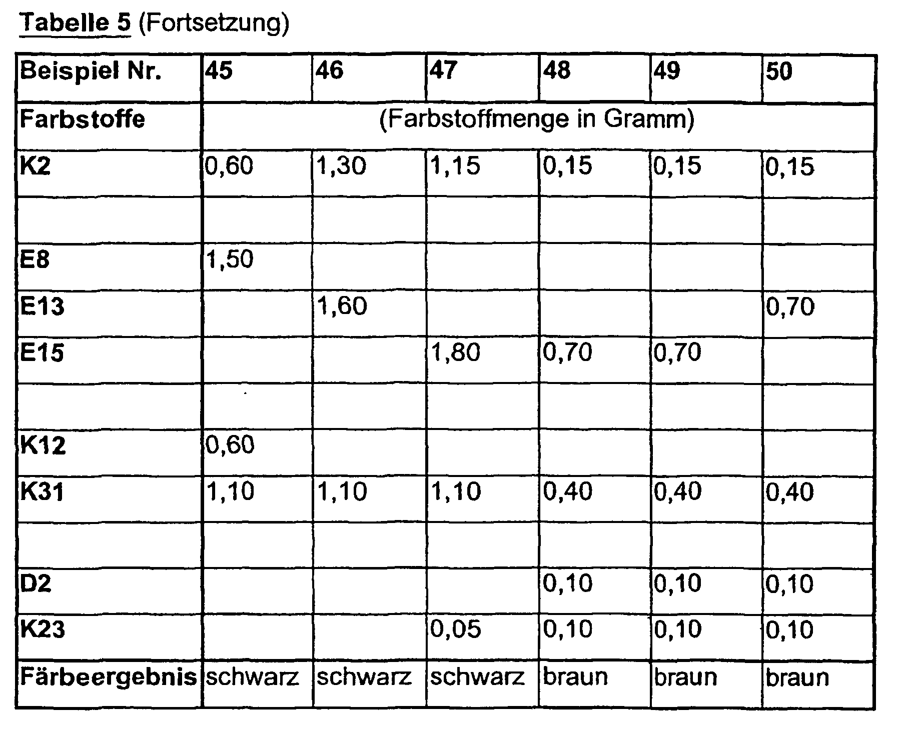

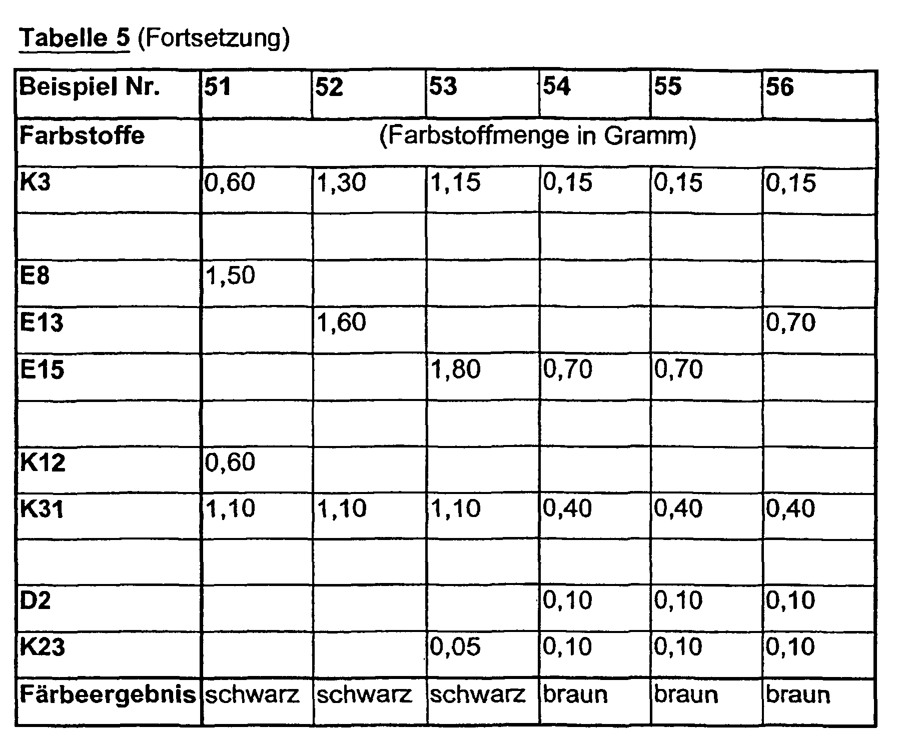

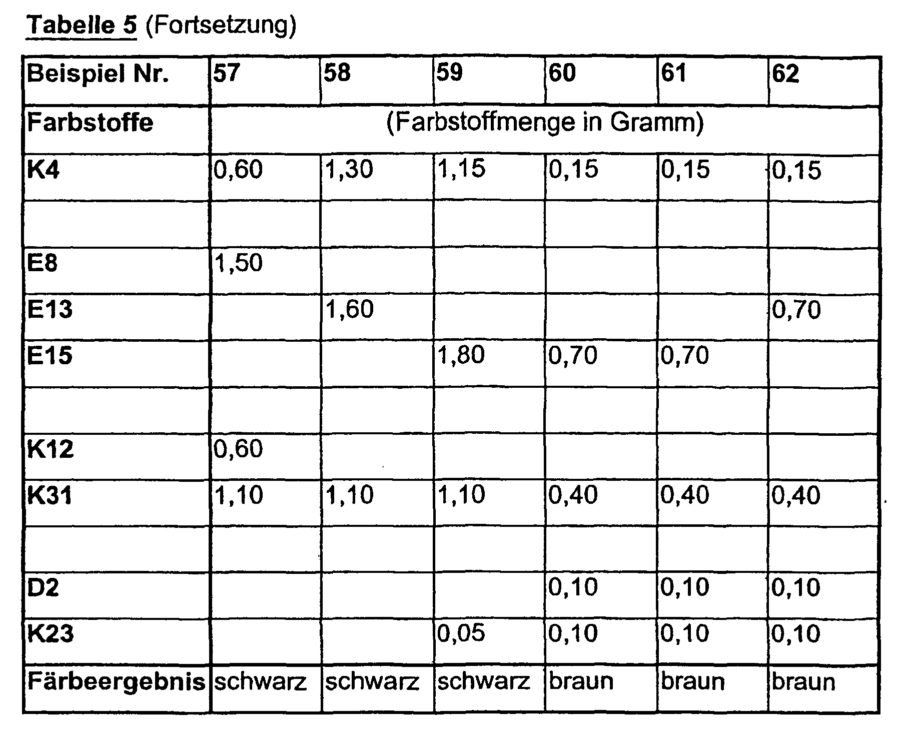

Alle in der vorliegenden Anmeldung enthaltenen Prozentangaben stellen -soweit nicht anders angegeben- Gewichtsprozente dar.

## Patentansprüche

1. Mittel zur oxidativen Färbung von Keratinfasem auf der Basis einer Entwicklersubstanz-Kupplersubstanz-Kombination, **dadurch gekennzeichnet, dass** es als Kupplersubstanz mindestens ein N-Heteroaryl-methyl-m-phenylendiamin-Derivat der Formel (I) oder dessen Salz mit einer organischen oder anorganischen Säure enthält, 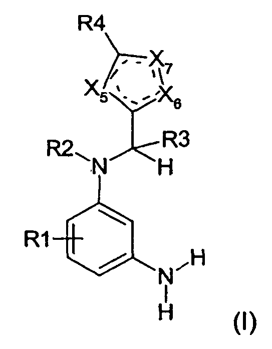 worin
**X5** gleich Schwefel, Stickstoff, Sauerstoff, C-R6 oder N-R5 ist;
**X6** gleich Schwefel, Stickstoff, Sauerstoff, C-R7 oder N-R5 ist
**X7** gleich Schwefel, Stickstoff, Sauerstoff, C-R8 oder N-R5 ist;
**R1** gleich Wasserstoff, einer C₁-C₄-Alkylgruppe, eine C₁-C₄-Hydroxyalkoxygruppe oder einer C₁-C₄-Hydroxyalkylgruppe ist ;
**R2** und **R3** gleich oder verschieden sein können und unabhängig voneinander gleich Wasserstoff oder einer C₁-C₆-Alkylgruppe sind;
**R4, R6, R7, R8** gleich oder verschieden sein können und unabhängig voneinander gleich Wasserstoff, einem Halogenatom (F, Cl, Br, J), einer Cyanogruppe, einer C₁-C₄-Alkoxygruppe, einer C₁-C₆-Alkylgruppe, einer C₁-C₄-Alkylthioethergruppe, einer Mercaptogruppe, einer Nitrogruppe, einer Aminogruppe, einer C₁-C₄-Alkylaminogruppe, einer Di(C₁-C₄)-alkylaminogruppe, einer Di(C₁-C₄-hydroxyalkyl)aminogruppe, einer C₁-C₄₋Hydroxyalkylaminogruppe, einer Trifluormethangruppe, einer -C(O)CH₃₋Gruppe, einer -C(O)CF₃-Gruppe, einer -Si(CH₃)₃-Gruppe, einer C₁-C₄₋Hydroxyalkylgruppe oder einer C₃-C₄-Dihydroxyalkylgruppe sind; und
**R5** gleich Wasserstoff, einer C₁-C₆-Alkylgruppe, einer C₂-C₄-Hydroxyalkylgruppe, einer Phenylgruppe oder einer Acetylgruppe ist; wobei mindestens einer und maximal zwei der Reste **X5** bis **X7** gleich C-R6 beziehungsweise C-R7 beziehungsweise C-R8 ist und maximal einer der Reste **X5** bis **X7** gleich Schwefel, Sauerstoff oder N-R5 ist.

2. Mittel nach Anspruch 1, **dadurch gekennzeichnet, dass R1** gleich Wasserstoff oder eine Hydroxyalkoxygruppe ist und die Reste **R2** und **R3** oder die Reste **R2**, **R3** und **R4** Wasserstoff bedeuten.

3. Mittel nach Anspruch 1 oder 2, **dadurch gekennzeichnet, dass R1** gleich Wasserstoff oder eine Hydroxyalkoxygruppe ist, die Reste **R2** und **R3** oder die Reste **R2, R3** und **R4** Wasserstoff bedeuten, **X5** gleich Schwefel oder Sauerstoff ist, **X6** gleich Stickstoff oder C-R7 ist und **X7** gleich C-R8 ist, wobei mindestens einer der Reste **R7** und **R8** gleich Wasserstoff ist.

4. Mittel nach Anspruch 1 oder 2, **dadurch gekennzeichnet, dass R1** gleich Wasserstoff oder eine Hydroxyalkoxygruppe ist, die Reste **R2** und **R3** oder die Reste **R2, R3** und **R4** gleich Wasserstoff sind, **X7** gleich Schwefel oder Sauerstoff ist, **X5** gleich C-R6 und **X6** gleich C-R7 ist, wobei mindestens einer der Reste **R6** und **R7** gleich Wasserstoff ist.

5. Mittel nach Anspruch 1 oder 2, **dadurch gekennzeichnet, dass R1** gleich Wasserstoff oder eine Hydroxyalkoxygruppe ist, die Reste **R2** und **R3** oder die Reste **R2, R3** und **R4** gleich Wasserstoff sind, **X6** gleich Schwefel oder Sauerstoff ist, **X5** gleich C-R6 und **X7** gleich C-R8 ist, wobei mindestens einer der Reste **R6** und **R8** gleich Wasserstoff ist.

6. Mittel nach einem der Ansprüche 1 bis 5, **dadurch gekennzeichnet, dass** das N-Heteroarylmethyl-m-phenylendiamin-Derivate der Formel (I) ausgewählt ist aus N-Thiophen-3-ylmethyl-1,3-diamino-benzol, N-Furan-3-ylmethyl-1,3-diamino-benzol, N-Furan-2-ylmethyl-1,3-diamino-benzol, N-Thiophen-2-ylmethyl-1,3-diamino-benzol, 2-{4-Amino-2-[(thiophen-3-ylmethyl)-amino]-phenoxy}-ethanol, 2-{4-Amino-2-[(thiophen-2-ylmethyl)-amino]-phenoxy}-ethanol, 2-{4-Amino-2-[(furan-3-ylmethyl)-amino]-phenoxy}-ethanol, 2-{4-Amino-2-[(furan-2-ylmethyl}-amino]-phenoxy}-ethanol, 2-{2-Amino-4-[(thiophen-2-ylmethyl)-amino]-phenoxy}-ethanol und 2-{2-Amino-4-[(furan-2-ytmethyl)-amino]-phenoxy}-ethanol oder deren Salzen.

7. Mittel nach einem der Ansprüche 1 bis 6, **dadurch gekennzeichnet, dass** das N-Heteroarylmethyl-m-phenylendiamin der Formel (I) in einer Menge von 0,005 bis 20 Gewichtsprozent enthalten ist.

8. Mittel nach einem der Ansprüche 1 bis 7, **dadurch gekennzeichnet, dass** die Entwicklersubstanz ausgewählt ist aus der Gruppe bestehend aus 1,4-Diaminobenzol, 1,4-Diamino-2-methyl-benzol, 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 1,4-Diamino-2-(pyridin-3-yl)benzol, 2,5-Diaminobiphenyl, 1,4-Diamino-2-methoxymethyl-benzol, 1,4-Diamino-2-aminomethyl-benzol, 1,4-Diamino-2-hydroxymethyl-benzol, 1,4-Diamino-2-(2-hydroxyethoxy)-benzol, 1-(2,5-Diamino-phenyl)-ethanol, 2-(2-(Acetyl-amino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)amino]-anilin, 4-[Di(2-hydroxyethyl)amino]-anilin, 4-[Di(2-hydroxyethyl)amino]-2-methyl-anilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(2-hydroxyethyl)-benzol, 1,4-Diamino-2-(1-methyl-ethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluor-phenol, 4-Methylamino-phenol, 4-Amino-2-( aminomethyl)-phenol, 4-Amino-2-(hydroxymethyl)-phenol, 4-Amino-2-fluor-phenol, 4-Amino-2-[(2-hydroxyethyl)-amino]methylphenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methyl-ethyl)-1H-pyrazol, 4,5-Diamino-1-[(4-methylphenyl)methyl]-1H-pyrazol, 1-[(4-Chlorphenyl)methyl]-4,5-diamino-1H-pyrazol, 4,5-Diamino-1-methyl-1H-pyrazol, 4,5-Diamino-3-methyl-1-methyl-1H-pyrazol, 4,5-Diamino-3-methyl-1-methyl-1H-pyrazol, 2-Amino-phenol, 2-Amino-6-methyl-phenol und 2-Amino-5-methyl-phenol.

9. Mittel nach einem der Ansprüche 1 bis 8, **dadurch gekennzeichnet, dass** es zusätzlich mindestens eine weitere Kupplersubstanz und/oder einen direktziehenden Farbstoff enthält.

10. Mittel nach einem der Ansprüche 1 bis 9, **dadurch gekennzeichnet, dass** die Entwicklersubstanzen und zusätzlichen Kupplersubstanzen, bezogen auf die Gesamtmenge des Färbemittels, jeweils in einer Gesamtmenge von 0,005 bis 20 Gewichtsprozent enthalten sind.

11. Mittel nach einem der Ansprüche 1 bis 10, **dadurch gekennzeichnet, dass** es vor der Anwendung mit dem Oxidationsmittel in einem Gewichtsverhältnis von 5:1 bis 1:3 vermischt wird.

12. Mittel nach Anspruch 11, **dadurch gekennzeichnet, dass** das gebrauchsfertige Oxidationsfärbemittel einen pH-Wert von 3 bis 11,5 aufweist.

13. Mittel nach einem der Ansprüche 1 bis 12, **dadurch gekennzeichnet, dass** es ein Haarfärbemittel ist.

14. N-Heteroaryl-methyl-m-phenylendiamin-Derivate der allgemeinen Formel (II) oder deren wasserlösliche Salze mit organischen oder anorganischen Säuren, 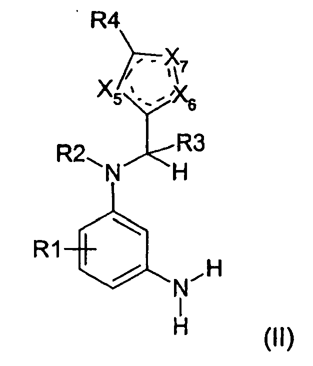 worin
worin
**X5** gleich Schwefel, Stickstoff, Sauerstoff, C-R6 oder N-R5 ist;
**X6** gleich Schwefel, Stickstoff, Sauerstoff, C-R7 oder N-R5 ist
**X7** gleich Schwefel, Stickstoff, Sauerstoff, C-R8 oder N-R5 ist;
**R1** gleich Wasserstoff, einer C₁-C₄-Alkylgruppe, eine C₁-C₄-Hydroxyalkoxygruppe oder einer C₁-C₄-Hydroxyalkylgruppe ist;
**R2** und **R3** gleich oder verschieden sein können und unabhängig voneinander gleich Wasserstoff oder einer C₁-C₆-Alkylgruppe sind;
**R4, R6, R7, R8** gleich oder verschieden sein können und unabhängig voneinander gleich Wasserstoff, einem Halogenatom (F, Cl, Br, J), einer Cyanogruppe, einer C₁-C₄-Alkoxygruppe, einer C₁-C₆-Alkylgruppe, einer C₁-C₄-Alkylthioethergruppe, einer Mercaptogruppe, einer Nitrogruppe, einer Aminogruppe, einer C₁-C₄-Alkylaminogruppe, einer Di(C₁-C₄)-alkylaminogruppe, einer Di(C₁-C₄-hydroxyalkyl)aminogruppe, einer C₁-C₄₋Hydroxyalkylaminogruppe, einer Trifluormethangruppe, einer -C(O)CH₃₋Gruppe, einer -C(O)CF₃-Gruppe, einer -Si(CH₃)₃-Gruppe, einer C₁-C₄₋Hydroxyalkylgruppe oder einer C₃-C₄-Dihydroxyalkylgruppe sind; und
**R5** gleich Wasserstoff, einer C₁-C₆-Alkylgruppe, einer C₂-C₁-Hydroxyalkylgruppe, einer Phenylgruppe oder einer Acetylgruppe ist; wobei mindestens einer und maximal zwei der Reste **X5** bis **X7** gleich C-R6 beziehungsweise C-R7 beziehungsweise C-R8 ist und maximal einer der Reste **X5** bis **X7** gleich Schwefel, Sauerstoff oder N-R5 ist;
unter den Bedingungen, dass mindestens einer der Reste **R1**, **R2, R3, R4, R6, R7** und **R8** nicht gleich Wasserstoff ist wenn **X5** oder **X6** gleich Schwefel ist.

## Claims

1. Agent for the oxidative dyeing of keratin fibres based on a developer substance-coupler substance combination, **characterized in that** it comprises, as coupler substance, at least one N-heteroaryl-methyl-m-phenylenediamine derivative of the formula (I) or salt thereof with an organic or inorganic acid, 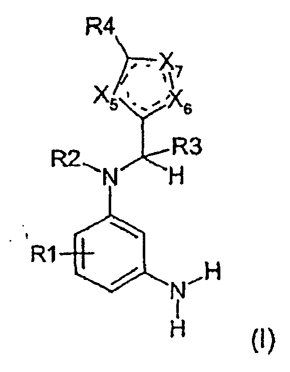 in which
**X5** is sulphur, nitrogen, oxygen, C-R6 or N-R5;
**X6** is sulphur, nitrogen, oxygen, C-R7 or N-R5;
**X7** is sulphur, nitrogen, oxygen, C-R8 or N-R5;
**R1** is hydrogen, a C₁-C₄-alkyl group, a C₁-C₄-hydroxyalkoxy group or a C₁-C₄-hydroxyalkyl group;
**R2** and **R3** may be identical or different and, independently of one another, are hydrogen or a C₁-C₆-alkyl group;
**R4, R6, R7, R8** may be identical or different and, independently of one another, are hydrogen, a halogen atom (F, Cl, Br, I), a cyano group, a C₁-C₄-alkoxy group, a C₁-C₆-alkyl group, a C₁-C₄-alkyl thioether group, a mercapto group, a nitro group, an amino group, a C₁-C₄-alkylamino group, a di (C₁-C₄) -alkylamino group, a di (C₁-C₄-hydroxyalkyl)amino-group, a C₁-C₄-hydroxyalkylamino group, a trifluoromethane group, a -C(O)CH₃ group, a -C(O)CF₃ group, a -Si(CH₃)₃ group, a C₁-C₄-hydroxyalkyl group or a C₃-C₄-dihydroxyalkyl group; and
**R5** is hydrogen, a C₁-C₆-alkyl group, a C₂-C₄-hydroxyalkyl group, a phenyl group or an acetyl group; where at least one and at most two of the radicals **X5** to **X7** is C-R6 or C-R7 or C-R8 and at most one of the radicals **X5** to **X7** is sulphur, oxygen or N-R5.

2. Agent according to Claim 1, **characterized in that R1** is hydrogen or a hydroxyalkoxy group and the radicals **R2** and **R3** or the radicals **R2, R3** and **R4** are hydrogen.

3. Agent according to Claim 1 or 2, **characterized in that R1** is hydrogen or a hydroxyalkoxy group, the radicals **R2** and **R3** or the radicals **R2, R3** and **R4** are hydrogen, **X5** is sulphur or oxygen, **X6** is nitrogen or C-R7 and **X7** is C-R8, where at least one of the radicals **R7** and **R8** is hydrogen.

4. Agent according to Claim 1 or 2, **characterized in that R1** is hydrogen or a hydroxyalkoxy group, the radicals **R2** and **R3** or the radicals **R2, R3** and **R4** are hydrogen, **X7** is sulphur or oxygen, **X5** is C-R6 and **X6** is C-R7, where at least one of the radicals **R6** and **R7** is hydrogen.

5. Agent according to Claim 1 or 2, **characterized in that R1** is hydrogen or a hydroxyalkoxy group, the radicals **R2** and **R3** or the radicals **R2, R3** and **R4** are hydrogen, **X6** is sulphur or oxygen, **X5** is C-R6 and **X7** is C-R8, where at least one of the radicals **R6** and **R8** is hydrogen.

6. Agent according to one of Claims 1 to 5, **characterized in that** the N-heteroarylmethyl-m-phenylenediamine derivative of the formula (I) is chosen from N-thiophen-3-ylmethyl-1,3-diaminobenzene, N-furan-3-ylmethyl-1,3-diaminobenzene, N-furan-2-ylmethyl-1,3-diaminobenzene, N-thiophen-2-ylmethyl-1,3-diaminobenzene, 2-{4-amino-2-[(thiophen-3-ylmethyl)amino]phenoxy}ethanol, 2-(4-amino-2-[(thiophen-2-ylmethyl)amino]phenoxy}ethanol, 2-{4-amino-2-[(furan-3-ylmethyl)amino]phenoxy}-ethanol, 2-{4-amino-2-[(furan-2-ylmethyl)amino]-phenoxy}ethanol, 2-{2-amino-4-[(thiophen-2-ylmethyl)amino]phenoxy}ethanol and 2-{2-amino-4-[(furan-2-ylmethyl)amino]phenoxyl}ethanol or salts thereof.

7. Agent according to one of Claims 1 to 6, **characterized in that** N-heteroarylmethyl-m-phenylenediamine of the formula (I) is present in an amount of from 0.005 to 20 per cent by weight.

8. Agent according to one of Claims 1 to 7, **characterized in that** the developer substance is chosen from the group consisting of 1,4-diaminobenzene, 1,4-diamino-2-methylbenzene, 1,4-diamino-2,6-dimethylbenzene, 1,4-diamino-3,5-diethylbenzene, 1,4-diamino-2,5-dimethylbenzene, 1,4-diamino-2,3-dimethylbenzene, 2-chloro-1,4-diaminobenzene, 1,4-diamino-2-(thiophen-2-yl)benzene, 1,4-diamino-2-(thiophen-3-yl)benzene, 1,4-diamino-2-(pyridin-3-yl)benzene, 2,5-diaminobiphenyl, 1,4-diamino-2-methoxymethylbenzene, 1,4-diamino-2-aminomethylbenzene, 1,4-diamino-2-hydroxymethylbenzene, 1,4-diamino-2-(2-hydroxyethoxy)benzene, 1-(2,5-diaminophenyl)ethanol, 2-(2-(acetylamino)ethoxy)-1,4-diaminobenzene, 4-phenylaminoaniline, 4-dimethylaminoaniline, 4-diethylaminoaniline, 4-dipropylaminoaniline, 4-[ethyl(2-hydroxyethyl)-amino]aniline, 9-[di(2-hydroxyethyl)amino]aniline, 4-[di(2-hydroxyethyl)amino]-2-methylaniline, 4-[(2-methoxyethyl)amino]aniline, 4-[(3-hydroxypropyl) amino] aniline, 4-[(2,3-dihydroxypropyl)-amino]aniline, 1,4-diamino-2-(2-hydroxyethyl)-benzene, 1,4-diamino-2-(1-methylethyl)benzene, 1,3-bis[(4-aminophenyl)(2-hydroxyethyl)amino]-2-propanol, 1,4-bis[(4-aminophenyl)amino]butane, 1,8-bis(2,5-diaminophenoxy)-3,6-dioxaoctane, 4-aminophenol, 4-amino-3-methylphenol, 4-amino-3-(hydroxymethyl)phenol, 4-amino-3-fluorophenol, 4-methylaminophenol, 4-amino-2-(aminomethyl)-phenol, 4-amino-2-(hydroxymethyl)phenol, 4-amino-2-fluorophenol, 4-amino-2-[(2-hydroxyethyl)amino]-methylphenol, 4-amino-2-methylphenol, 4-amino-2-(methoxymethyl)phenol, 4-amino-2-(2-hydroxyethyl)phenol, 5-aminosalicylic acid, 2,5-diaminopyridine, 2,4,5,6-tetraaminopyrimidine, 2,5,6-triamino-4-(1H)-pyrimidone, 4,5-diamino-1-(2-hydroxyethyl)-1H-pyrazole, 4,5-diamino-1-(1-methylethyl)-1H-pyrazole, 4,5-diamino-1-[(4-methylphenyl)-methyl]-1H-pyrazole, 1-[(4-chlorophenyl)methyl]-4,5-diamino-1H-pyrazole, 4,5-diamino-1-methyl-1H-pyrazole, 4,5-diamino-3-methyl-1-methyl-1H-pyrazole, 4,5-diamino-3-methyl-1-methyl-1H-pyrazole, 2-aminophenol, 2-amino-6-methylphenol and 2-amino-5-methylphenol.

9. Agent according to one of Claims 1 to 8, **characterized in that** it additionally comprises at least one further coupler substance and/or a direct dye.

10. Agent according to one of Claims 1 to 9, **characterized in that** the developer substances and additional coupler substances, based on the total amount of the dye, are in each case present in a total amount of from 0.005 to 20 per cent by weight.

11. Agent according to one of Claims 1 to 10, **characterized in that**, prior to application, it is mixed with the oxidizing agent in a weight ratio of from 5:1 to 1:3.

12. Agent according to Claim 11, **characterized in that** the ready-to-use oxidation dye has a pH of from 3 to 11.5.

13. Agent according to one of Claims 1 to 12, **characterized in that** it is a hair dye.

14. N-Heteroarylmethyl-m-phenylenediamine derivatives of the general formula (II) or water-soluble salts thereof with organic or inorganic acids, 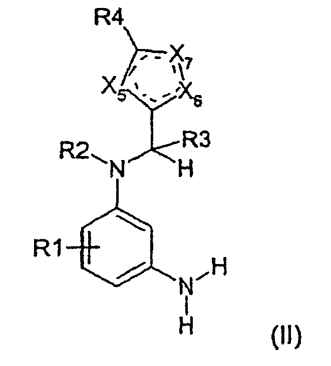 in which
**X5** is sulphur, nitrogen, oxygen, C-R6 or N-R5;
**X6** is sulphur, nitrogen, oxygen, C-R7 or N-R5;
**X7** is sulphur, nitrogen, oxygen, C-R8 or N-R5;
**R1** is hydrogen, a C₁-C₄-alkyl group, a C₁-C₄-hydroxyalkoxy group or a C₁-C₄-hydroxyalkyl group;
**R2** and **R3** may be identical or different and, independently of one another, are hydrogen or a C₁-C₆-alkyl group;
**R4, R6, R7, R8** may be identical or different and, independently of one another, are hydrogen, a halogen atom (F, Cl, Br, I), a cyano group, a C₁-C₄-alkoxy group, a C₁-C₆-alkyl group, a C₁-C₄-alkyl thioether group, a mercapto group, a nitro group, an amino group, a C₁-C₄-alkylamino group, a di (C₁-C₄) -alkylamino group, a di (C₁-C₄-hydroxyalkyl)amino group, a C₁-C₄-hydroxyalkylamino group, a trifluoromethane group, a -C(O)CH₃ group, a -C(O)CF₃ group, a -Si(CH₃)₃ group, a C₁-C₄-hydroxyalkyl group or a C₃-C₄-dihydroxyalkyl group; and
**R5** is hydrogen, a C₁-C₆-alkyl group, a C₂-C₄-hydroxyalkyl group, a phenyl group or an acetyl group; where at least one and at most two of the radicals **X5** to **X7** is C-R6 or C-R7 or C-R8 and at most one of the radicals **X5** to **X7** is sulphur, oxygen or N-R5;
under the conditions that at least one of the radicals **R1, R2, R3, R4, R6, R7** and **R8** is not hydrogen if **X5** or **X6** is sulphur.

## Revendications

1. Composition pour la teinture par oxydation de fibres de kératine, à base d'une association développeur-coupleur, **caractérisée en ce qu'**elle contient en tant que coupleur au moins un dérivé de N-hétéroarylméthyl-m-phénylènediamine de formule (I) ou un sel d'un tel dérivé avec un acide organique ou minéral, 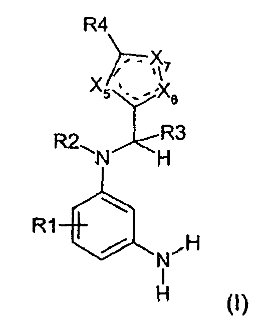 formule dans laquelle
**X5** représente un atome de soufre, d'azote ou d'oxygène, C-R6 ou N-R5 ;
**X6** représente un atome de soufre, d'azote ou d'oxygène, C-R7 ou N-R5 ;
**X7** représente un atome de soufre, d'azote ou d'oxygène, C-R8 ou N-R5 ;
**R1** représente un atome d'hydrogène, un groupe alkyle en C₁-C₄, un groupe hydroxyalcoxy en C₁-C₄ ou un groupe hydroxyalkyle en C₁-C₄ ;
**R2** et **R3** peuvent être identiques ou différents et représentent, indépendamment l'un de l'autre, un atome d'hydrogène ou un groupe alkyle en C₁-C₆ ;
**R4, R6, R7, R8** peuvent être identiques ou différents et représentent, indépendamment les uns des autres, un atome d'hydrogène, un atome d'halogène (F, Cl, Br, I), un groupe cyano, un groupe alcoxy en C₁-C₄, un groupe alkyle en C₁-C₆, un groupe alkyl(C₁-C₄)thioéther, un groupe mercapto, un groupe nitro, un groupe amino, un groupe alkyl (C₁-C₄) amino, un groupe dialkyl (C₁-C₄) amino, un groupe di[hydroxyalkyl (C₁-C₄)] amino, un groupe hydroxyalkyl-(C₁-C₄) amino, un groupe trifluorométhane, un groupe -C-(O)CH₃, un groupe -C(O)CF₃, un groupe -Si(CH₃)_{3'} un groupe hydroxyalkyle en C₁-C₄ ou un groupe dihydroxyalkyle en C₃-C₄ ; et
**R5** représente un atome d'hydrogène, un groupe alkyle en C₁-C₆, un groupe hydroxyalkyle en C₂-C₄, un groupe phényle ou un groupe acétyle ;
au moins un et au maximum deux des radicaux **X5** à **X7** représentant C-R6 ou C-R7 ou C-R8 et au maximum un des radicaux **X5** à **X7** étant un atome de soufre ou d'oxygène ou N-R5.

2. Composition selon la revendication 1, **caractérisée en ce que R1** est un atome d'hydrogène ou un groupe hydroxyalcoxy et les radicaux **R2** et **R3** ou les radicaux **R2, R3** et **R4** représentent des atomes d'hydrogène.

3. Composition selon la revendication 1 ou 2, **caractérisée en ce que R1** est un atome d'hydrogène ou un groupe hydroxyalcoxy, les radicaux **R2** et **R3** ou les radicaux **R2, R3** et **R4** représentent des atomes d'hydrogène, **X5** est un atome de soufre ou d'oxygène, **X6** est un atome d'azote ou est égal à C-R7 et **X7** est C-R8, au moins l'un des radicaux **R7** et R8 étant égal à un atome d'hydrogène.

4. Composition selon la revendication 1 ou 2, **caractérisée en ce que R1** est un atome d'hydrogène ou un groupe hydroxyalcoxy, les radicaux **R2** et **R3** ou les radicaux **R2, R3** et **R4** représentent des atomes d'hydrogène, **X7** est un atome de soufre ou d'oxygène, **X5** est égal à C-R6 et **X6** est égal à C-R7, au moins l'un des radicaux **R6** et **R7** étant un atome d'hydrogène.

5. Composition selon la revendication 1 ou 2, **caractérisée en ce que R1** est un atome d'hydrogène ou un groupe hydroxyalcoxy, les radicaux **R2** et **R3** ou les radicaux **R2, R3** et **R4** représentent des atomes d'hydrogène, **X6** est un atome de soufre ou d'oxygène, **X5** est égal à C-R6 et **X7** est égal à C-R8, au moins l'un des radicaux **R6** et **R8** étant un atome d'hydrogène.

6. Composition selon l'une quelconque des revendications 1 à 5, **caractérisée en ce que** le dérivé de N-hétéroarylméthyl-m-phénylènediamine de formule (I) est choisi parmi le N-thiophén-3-ylméthyl-1,3-diaminobenzène, le N-furan-3-ylméthyl-1,3-diaminobenzène, le N-furan-2-ylméthyl-1,3-diaminobenzène, le N-thiophén-2-ylméthyl-1,3-diaminobenzène, le 2-{4-amino-2-[(thiophén-3-ylméthyl)-amino]-phénoxy}-éthanol, le 2-{4-amino-2-[(thiophén-2-ylméthyl)-amino]-phénoxy}-éthanol, le 2-{4-amino-2-[(furan-3-ylméthyl)-amino]-phénoxy}-éthanol, le 2-{4-amino-2-[(furan-2-ylméthyl)-amino]-phénoxy}-éthanol, le 2-{2-amino-4-[(thiophén-2-ylméthyl)-amino]-phénoxy}-éthanol et le 2-{2-amino-4-[(furan-2-ylméthyl)-amino]-phénoxy}-éthanol ou leurs sels.

7. Composition selon l'une quelconque des revendications 1 à 6, **caractérisée en ce que** la N-hétéroarylméthyl-m-phénylènediamine de formule (I) est contenue en une quantité de 0,005 à 20 % en poids.

8. Composition selon l'une quelconque des revendications 1 à 7, **caractérisée en ce que** le développeur est choisi dans le groupe constitué par le 1,4-diaminobenzène, le 1,4-diamino-2-méthylbenzène, le 1,4-diamino-2,6-diméthylbenzène, le 1,4-diamino-3,5-diéthylbenzène, le 1,4-diamino-2,5-,diméthylbenzène, le 1,4-diamino-2,3-diméthylbenzène, le 2-chloro-1,4-diaminobenzène, le 1,4-diamino-2-(thiophén-2-yl)benzène, le 1,4-diamino-2-(thiophén-3-yl)benzène, le 1,4-diamino-2-(pyridin-3-yl)-benzène, le 2,5-diaminobiphényle, le 1,4-diamino-2-méthoxyméthylbenzène, le 1,4-diamino-2-aminométhylbenzène, le 1,4-diamino-2-hydroxyméthylbenzène, le 1,4-diamino-2-(2-hydroxyéthoxy)benzène, le 1-(2,5-diamino-phényl)éthanol, le 2-(2-(acétylamino)-éthoxy)-1,4-diaminobenzène, la 4-phénylaminoaniline, la 4-diméthylamino-aniline, la 4-diéthylamino-aniline, la 4-dipropylamino-aniline, la 4-[éthyl-(2-hydroxyéthyl)amino]aniline, la 4-[di(2-hydroxyéthyl)amino]aniline, la 4- [di (2-hydroxyéthyl)amino]-2-méthylaniline, la 4-[(2-méthoxyéthyl)amino]aniline, la 4-[(3-hydroxypropyl)amino]aniline, la 4-[(2,3-dihydroxypropyl)amino]aniline, le 1,4-diamino-2-(2-hydroxyéthyl) benzène, le 1,4-diamino-2-(1-méthyléthyl)-benzène, le 1,3-bis[(4-aminophényl)(2-hydroxyéthyl)amino]-2-propanol, le 1,4-bis[(4-aminophényl)amino]butane, le 1,8-bis(2,5-diaminophénoxy)-3,6-dioxaoctane, le 4-aminophénol, le 4-amino-3-méthylphénol, le 4-amino-3-(hydroxyméthyl)phénol, le 4-amino-3-fluorophénol, le 4-méthylaminophénol, le 4-amino-2-(aminométhyl)-phénol, le 4-amino-2-(hydroxyméthyl)phénol, le 4-amino-2-fluorophénol, le 4-amino-2-[(2-hydroxyéthyl) amino] méthylphénol, le 4-amino-2-méthylphénol, le 4-amino-2-(méthoxyméthyl)phénol, le 4-amino-2-(2-hydroxyéthyl)phénol, l'acide 5-aminosalicylique, la 2,5-diaminopyridine, la 2,4,5,6-tétraaminopyrimidine, la 2,5,6-triamino-4(1H)-pyrimidone, le 4,5-diamino-1-(2-hydroxyéthyl)-1H-pyrazole, le 4,5-diamino-1-(1-méthyléthyl)-1H-pyrazole, le 4,5-diamino-1-[(4-méthylphényl)méthyl]-1H-pyrazole, le 1-[(4-chlorophényl)-méthyl]-4,5-diamino-1H-pyrazole, le 4,5-diamino-1-méthyl-1H-pyrazole, le 4,5-diamino-3-méthyl-1-méthyl-1H-pyrazole, le 4,5-diamino-3-méthyl-1-méthyl-1H-pyrazole, le 2-aminophénol, le 2-amino-6-méthylphénol et le 2-amino-5-méthylphénol.

9. Composition selon l'une quelconque des revendications 1 à 8, **caractérisée en ce qu'**en outre elle contient au moins un autre coupleur et/ou un colorant direct.

10. Composition selon l'une quelconque des revendications 1 à 9, **caractérisée en ce que** les développeurs et les coupleurs supplémentaires sont contenus chacun, par rapport à la quantité totale de la composition de teinture, en une quantité totale de 0,005 à 20 % en poids.

11. Composition selon l'une quelconque des revendications 1 à 10, **caractérisée en ce qu'**avant l'emploi on la mélange avec l'oxydant en un rapport pondéral de 5:1 à 1:3.

12. Composition selon la revendication 11, **caractérisée en ce que** la composition de teinture par oxydation présente un pH de 3 à 11,5.

13. Composition selon l'une quelconque des revendications 1 à 12, **caractérisée en ce qu'**il s'agit d'une composition de teinture pour cheveux.

14. Dérivés de N-hétéroarylméthyl-m-phénylènediamine de formule générale (II) ou sels hydrosolubles avec des acides organiques ou minéraux de tels dérivés, 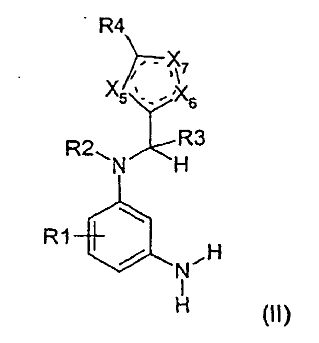 formule dans laquelle
**X5** représente un atome de soufre, d'azote ou d'oxygène, C-R6 ou N-R5 ;
**X6** représente un atome de soufre, d'azote ou d'oxygène, C-R7 ou N-R5 ;
**X7** représente un atome de soufre, d'azote ou d'oxygène, C-R8 ou N-R5 ;
**R1** représente un atome d'hydrogène, un groupe alkyle en C₁-C₄, un groupe hydroxyalcoxy en C₁-C₄ ou un groupe hydroxyalkyle en C₁-C₄ ;
**R2** et **R3** peuvent être identiques ou différents et représentent, indépendamment l'un de l'autre, un atome d'hydrogène ou un groupe alkyle en C₁-C₆ ;
**R4, R6, R7, R8** peuvent être identiques ou différents et représentent, indépendamment les uns des autres, un atome d'hydrogène, un atome d'halogéne (F, Cl, Br, I), un groupe cyano, un groupe alcoxy en C₁-C₄, un groupe alkyle en C₁-C₆, un groupe alkyl(C₁-C₄)thioéther, un groupe mercapto, un groupe nitro, un groupe amino, un groupe alkyl (C₁-C₄) amino, un groupe dialkyl(C₁-C₄)amino, un groupe di [hydroxyalkyl(C₁-C₄)]amino, un groupe hydroxyalkyl-(C₁-C₄)amino, un groupe trifluorométhane, un groupe -C-(O)CH₃, un groupe -C(O)CF₃, un groupe -Si(CH₃)_{3'} un groupe hydroxyalkyle en C₁-C₄ ou un groupe dihydroxyalkyle en C₃-C₄ ; et
**R5** représente un atome d'hydrogène, un groupe alkyle en C₁-C₆, un groupe hydroxyalkyle en C₂-C₄. un groupe phényle ou un groupe acétyle ;
au moins un et au maximum deux des radicaux **X5** à **X7** représentant C-R6 ou C-R7 ou C-R8 et au maximum un des radicaux **X5** à **X7** étant un atome de soufre ou d'oxygène ou N-R5 ;
avec les conditions qu'au moins l'un des radicaux **R1, R2, R3, R4, R6, R7** et **R8** soit différent d'un atome d'hydrogène lorsque **X5** ou **X6** représente un atome de soufre.
